(19) Europäisches Patentamt
European Patent Office
Office européen des brevets

(11) Publication number: **0 095 666 B1**

(12) # EUROPEAN PATENT SPECIFICATION

(45) Date of publication of patent specification: **18.01.89**

(21) Application number: **83104932.5**

(22) Date of filing: **19.05.83**

(51) Int. Cl.[4]: **C 07 D 209/62,** C 07 D 405/06, C 07 D 209/66, C 07 C 121/48, A 61 K 31/40, C 07 C 121/75

(54) **Tetrahydro-benzo(e) isoindolines.**

(30) Priority: **01.06.82 US 383631**

(43) Date of publication of application: **07.12.83 Bulletin 83/49**

(45) Publication of the grant of the patent: **18.01.89 Bulletin 89/03**

(84) Designated Contracting States: **AT BE CH DE FR GB IT LI LU NL SE**

(56) References cited:
**EP-A-0 003 757**
**EP-A-0 029 581**
**US-A-3 166 570**

**Chemical Abstracts vol. 77, no. 25, 18 December 1972, Columbus, OH (US); K.D.PAULL et al.: "Facile synthesis of 4-substituted 3a, 4, 5, 9b-tetrahydrobenz(e)isoindoline", p. 387, no. 164371n**

**The file contains technical information submitted after the application was filed and not included in this specification**

(73) Proprietor: **ABBOTT LABORATORIES**
**14th Street and Sheridan Road North St**
**North Chicago Illinois 60064 (US)**

(72) Inventor: **DeBernardis, John Francis**
**35 Burnett Avenue**
**Lake Villa, IL 60046 (US)**
Inventor: **Kerkman, Daniel Joseph**
**36763 Grandwood Drive**
**Gurnee, IL 60031 (US)**
Inventor: **McClellan, William John**
**110 N. Park Avenue**
**Waukegan, IL 60085 (US)**

(74) Representative: **Modiano, Guido et al**
**MODIANO, JOSIF, PISANTY & STAUB Modiano & Associati Via Meravigli, 16**
**I-20123 Milan (IT)**

(56) References cited:
**Chemical Abstracts vol. 82, no. 25, 23 June 1975, Columbus, OH (US); L.A.WALTER et al.: "1,5-Ethane-2,3,4,5-tetrahydro-1H-3-benzazepines", p. 23, no. 164804j**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 666 B1

(56) References cited:
THE JOURNAL OF ORGANIC CHEMISTRY, vol. 46, no. 11, 22 May 1981, Columbus, OH (US); S.AMIN et al.: "Synthesis of angular -5-methylchrysenes and 5-methylchrysenols", pp. 2394-2398

## Description

This invention relates to novel tetrahydrobenzo[e]isoindolines useful in the treatment of hypertension.

The adrenergic nervous system plays a major role in the innervation of heart, blood vessel and smooth muscle tissue. Agents capable of interacting with receptor sites within the adrenergic nervous system can result in a variety of physiological responses, including vasoconstriction, vasodilation, and increased or decreased heart rate (chronotropic), contractility (inotropic) and metabolic activity. In the past, various adrenergic agents have been employed to affect these and other physiological responses. However, it is highly desirable to obtain new adrenergic agents which demonstrate a high degree of specificity for differing receptor types within the adrenergic nervous system in order to obtain a desired physiological response separate from other possible, and perhaps less desirable, responses of the system. This property has been lacking from most previously employed adrenergic agents. Thus, the search continues for new and improved adrenergic agents capable of selective interaction with adrenergic receptor sites.

Known from pages 3374—3376 of vol. 37(21) of the Journal of Organic Chemistry (1972), is the facile synthesis of 4 substituted 3a,4,5,9b-tetrahydrobenz[e]isoindolines, which were prepared from substituted 1 cyanobenzocyclobutenes via the corresponding tetrahydrobenz[e]isoindolin-3-ones.

US—A—3 166 570 teaches derivatives of benzo[e]isoindoline and their preparation. 5-phenyl-2-methyl-3a,4,5,9b-tetrahydrobenzo(e)isoindolines are described which are pharmacologically active and exhibit properties characteristic of tranquilizers and antidepressant drugs.

Also known from pages 2394—2398 of vol. 46(11) of the Journal of Organic Chemistry (1981), is the synthesis of substituted Chrysenes and Chrysenols, wherein 1-Cyan-6 methoxy-3.4 dihydro-naphthaline is described.

However, none of these above-cited prior art references deals with the problem of providing compounds which are useful as therapeutic agents in the treatment of hypertension.

EP—A—0 029 581 describes antihypertensive compounds, which however have a structure which is considerably different from that of the antihypertensive compounds which are described and claimed hereinafter.

An article by Lewis A. Walter et al. published in Journal of Medicinal Chemistry, 1975, Vol. 18, 206—208 (1975) discloses 1,5-ethano-2,3,4,5-tetrahydro-1H-3-benzazepines unsubstituted in the benzo ring tested for analgetic activity.

It has now been determined that a new class of compounds, the tetrahydro-benzo[e]isoindolines, as herein defined, demonstrate an ability to interact specifically with various adrenergic receptor types and are useful as therapeutic agents in the treatment of hypertension.

The present invention provides a compound of the formula

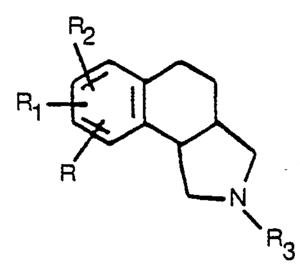

wherein R, $R_1$, and $R_2$ are independently selected from hydrogen, hydroxy, alkoxy of 1 to 3 carbon atoms, or R and $R_1$, or $R_1$ and $R_2$ can be taken together to form a methylenedioxy or ethylenedioxy bridge; with the proviso that at least one of R, $R_1$ or $R_2$ must be other than hydrogen; and $R_3$ is hydrogen; alkyl of 1 to 4 carbon atoms; a substituent of the formula

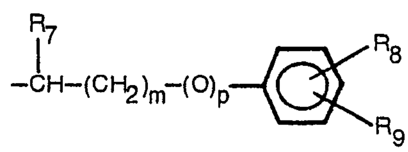

wherein m is 0, 1 or 2, p is 0 or 1, $R_7$ is hydrogen or alkyl of 1 to 4 carbon atoms and $R_8$ and $R_9$ are independently selected from hydrogen, hydroxy, methoxy, alkyl of 1 to 4 carbon atoms, or halo, or $R_8$ and $R_9$ can be taken together to form a methylenedioxy or ethylenedioxy bridge; or 1,4-benzodioxan of the formula

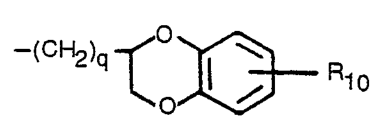

wherein q is 1, 2 or 3, and $R_{10}$ is hydrogen, methoxy, or halo; and the pharmaceutically acceptable salts thereof.

In a presently particularly preferred embodiment of the inventive concepts, the compounds of formula I are the substituted and unsubstituted tetrahydrodihydroxy-benzo[e]isoindolines and their pharmaceutically acceptable salts, such as 3a,4,5,9b-tetrahydro-6,7-dihydroxybenzo[e]isoindoline, 2-methyl-3a,4,5,9b-tetrahydro-6,7-dihydroxybenzo[e]isoindoline, 2-n-propyl-3a,4,5,9b-tetrahydro-6,7-dihydroxybenzo[e]isoindoline, 3a,4,5,9b-tetrahydro-7,8-dihydroxybenzo[e]isoindoline, 2-methyl-3a,4,5,9b-tetrahydro-7,8-dihydroxybenzo[e]isoindoline, 2-n-propyl-3a,4,5,9b-tetrahydro-7,8-dihydroxybenzo[e]isoindoline.

As used herein, the term "loweralkyl of 1 to 4 carbon atoms" means straight or branched chain saturated hydrocarbon radicals, such as methyl, ethyl, *n*-propyl, *iso*-propyl, *n*-butyl, *s*-butyl, and *t*-butyl. The term additionally includes halo-substituted loweralkyl groups such as, for example, trifluoromethyl, 2-trichloroethyl, and the like.

As used herein, the term "halo" means chloro, bromo, fluoro and iodo.

The term "pharmaceutically acceptable salts" refers to the pharmaceutically acceptable, relatively nontoxic, inorganic or organic acid addition salts of the compounds of this invention. These salts can be prepared *in situ* during the final isolation and purification of the compounds, or by separately reacting the free base with a suitable organic or inorganic acid. Representative salts include the hydrochloride, hydrobromide, sulfate, phosphate, nitrate, bisulfate, acetate, oxalate, valerate, oleate, palmitrate, stearate, laurate, borate, benzoate, lactate, phosphate, tosylate, citrate, maleate, fumarate, succinate, tartrate, napsylate. It will be apparent to those skilled in the art that, depending upon the number of available amino groups for salt formation, the salt of this invention can be per-*N*-salts.

The alkoxy- and hydroxy-substituted isoindolines of the invention may be obtained in accordance with the following reaction scheme:

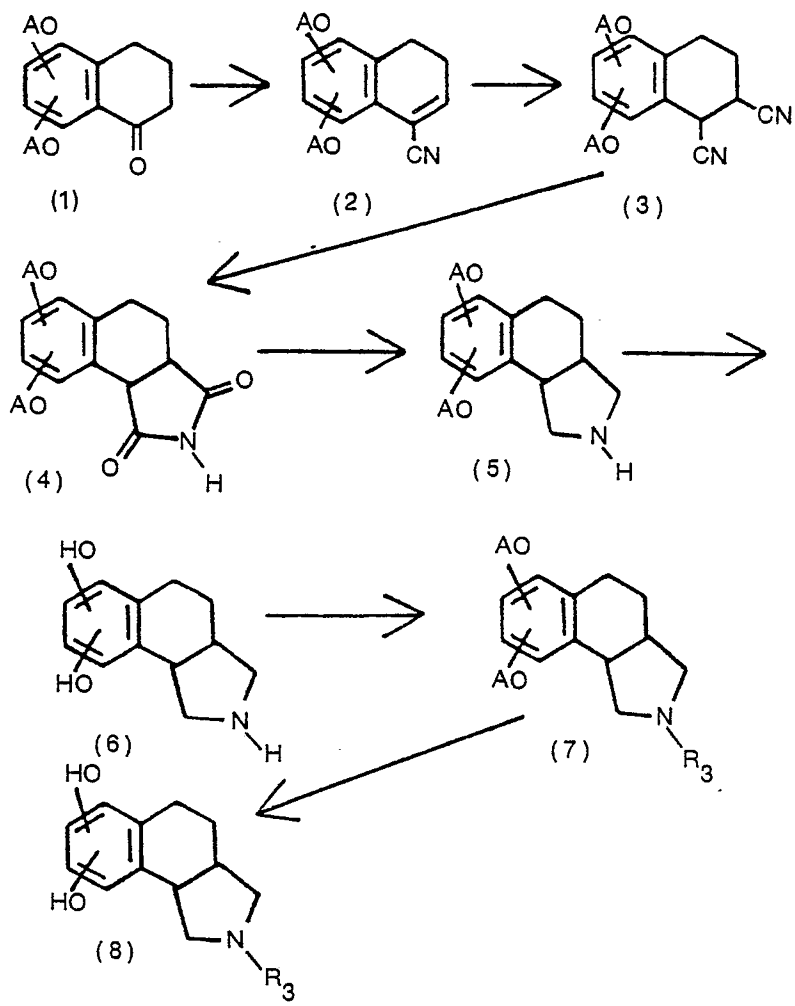

(1) (2) (3) (4) (5) (6) (7) (8)

wherein A is loweralkyl. In accordance with the foregoing reaction scheme, an alkoxy-substituted tetralone (1) is treated with trimethylsilylcyanide and a catalytic amount of aluminum trichloride, followed by treatment in methanol with hydrochloric acid to obtain the corresponding 1-cyano-3,4-dihydronaphthalene (2). To a suspension of the latter in methanol and diethyl ether is added potassium cyanide to obtain the corresponding 1,2-dicyano-1,2,3,4-tetrahydronaphthalene compound (3), which is suspended in ethanol and treated with sulfuric acid to yield the 1,3-dioxo-3a,4,5,9b-tetrahydro-benzo[e]isoindoline (4).

Alternatively, the tetrahydro-benzo[e]isoindoline (4) may be obtained by treating the 1,2-dicyano compound (3) in methylene dichloride with hydrogen bromide gas at a reduced temperature, followed by treatment with an aqueous solution of dimethylformamide. The 1,3-dioxo compound (4) is then reduced, such as with a complex of borane in tetrahydrofuran (THF) followed by methanolic hydrochloric acid, to obtain the 3a,4,5,9b-tetrahydro-benzo[e]isoindoline (5).

The alkoxy-substituted 3a,4,5,9b-tetrahydrobenzo[e]isoindoline (5) may be dealkylated, such as by treatment with boron tribromide, to obtain the corresponding hydroxy-substituted 3a,4,5,9b-tetrahydro-benzo[e]isoindoline (6).

The N-substituted compounds of the invention may be obtained by known amine alkylation and/or acylation techniques. For example, N-alkylated and N-arylalkylated compounds of the invention may be obtained by reacting a 3a,4,5,9b-tetrahydro-benzo[e]isoindoline (5) with an alkyl halide, such as 1-bromopropane, to obtain, for example, the corresponding 2-n-propyl compound, etc., or with an arylalkyl halide, such as benzyl chloride, to obtain, for example, the corresponding 2-benzyl compound. As a further example, a 3a,4,5,9b-tetrahydro-benzo[e]isoindoline (5) may be reacted with an acid chloride of a carboxylic or sulfonic acid to form the corresponding amide, which may then be reduced, such as with borane/THF.

The foregoing may be better understood in connection with the following examples:

Example 1

1-cyano-5,6-dimethoxy-3,4-dihydronaphthalene

A solution of 30 g of 5,6-dimethoxytetralone, 15.8 grams of trimethylsilylcyanide, 15 ml of dry benzene, and a catalytic amount of $AlCl_3$ is stirred under a nitrogen atmosphere in an oil bath at 70°C for 15 hours. Benzene is removed from the reaction mixture under vacuum. To the residue is added 300 ml of methanol, and the mixture is stirred at a temperature of 0° to 10°C as hydrochloric acid is bubbled through the mixture for a period of 3 hours. The mixture is evaporated under vacuum to remove methanol, and the residue is washed twice with water and then dried to yield 29.2 grams of 1-cyano-5,6-dimethoxy-3,4-dihydronaphthalene as a yellow solid, m.p. 139—140°C.

Example 2

1,2-dicyano-5,6-dimethoxy-1,2,3,4-tetrahydronaphthalene

A suspension of 29.2 grams of 1-cyano-5,6-dimethoxy-3,4-dihydronaphthalene, 315 ml of methanol, and 110 ml of diethyl ether is stirred at a temperature just below reflux while a solution of 34 grams of potassium cyanide in 94 ml of water at 45°C is added dropwise. The reaction mixture is stirred at reflux for 1.5 hours. After cooling briefly, a solution of 21 grams of ammonium acetate in 34 ml of water is added to the reaction mixture. Additional water is added to the reaction mixture until the mixture becomes cloudy, and the reaction mixture is stored at 0°C for 2 days. The solid phase is filtered from the reaction mixture, washed with water at 60°C, and then washed with cold 50% aqueous methanol. The solid phase is dried under vacuum to yield 14.0 grams of 1,2-dicyano-5,6-dimethoxy-1,2,3,4-tetrahydronaphthalene as a tan solid, m.p. 107—108°C.

Example 3

1,3-dioxo-3a,4,5,9b-tetrahydro-6,7-dimethoxybenzo[e]isoindoline

A slurry is prepared from 15.1 grams of 1,2-dicyano-5,6-dimethoxy-1,2,3,4-tetrahydronaphthalene and 30 ml of 95% aqueous ethanol and stirred at 50°C. To this slurry is added dropwise 13.5 ml of concentrated sulfuric acid, and the mixture is stirred at reflux for 0.5 hours, at which time the mixture solidifies. Heating is continued for an additional 1 hour, the mixture is cooled to room temperature and then 200 ml of water are added to the mixture. The mixture is stirred for 0.5 hours and then filtered. Solid residue is washed with two 100-ml portions of water, with sodium carbonate, and finally with water. The solid residue is dried under vacuum to yield 8.23 grams of a tan solid. The crude product is boiled with 4 portions of 150-ml of tetrane, decanting after each treatment. The tetrane insoluble solid is recrystallized from benzene/ethyl acetate to yield 4.2 grams of 1,3-dioxo-3a,4,5,9b-tetrahydro-6,7-dimethoxybenzo[e]isoindoline as a white solid, m.p. 220—221°C.

Example 4

1,3-dioxo-2-benzyl-3a,4,5,9b-tetrahydro-6,7-dimethoxybenzo[e]isoindoline

To a slurry of 4.1 grams of 1,3-dioxo-3a,4,5,9b-tetrahydro-6,7-dimethoxybenzo[e]isoindoline and 70 ml of dry dimethylformamide (DMF) stirred under a nitrogen atmosphere at room temperature, is added in portions a mixture of 0.83 grams of sodium hydride (50% in oil, washed with tetrane) and 8 ml of dry DMF. The reaction mixture is stirred at room temperature for 1.5 hours. 2.2 grams of benzylchloride is added to the reaction mixture and the mixture is heated at 55—60°C for 2 hours. After cooling to room temperature the solution is poured onto 300 ml of ice water while stirring. The mixture is stirred for 0.5 hours, the solid is filtered, washed with water, and dried under vacuum to yield 5.3 grams of a yellow solid. The crude product is washed with boiling tetrane and filtered. The residue is dried under vacuum overnight to obtain 4.6 grams of 1,3-dioxo-2-benzyl-3a,4,5,9b-tetrahydro-6,7-dimethoxybenzo[e]isoindoline as a yellow solid, m.p. 167—169°C.

Example 5

2-benzyl-3a,4,5,9b-tetrahydro-6,7-dimethoxybenzo[e]isoindoline hydrochloride

65 ml of borane (1 *M*) in tetrahydrofuran (THF) is stirred under a nitrogen atmosphere at 0°C as a solution of 4.5 grams of 1,3-dioxo-2-benzyl-3a,4,5,9b-tetrahydro-6,7-dimethoxybenzo[e]isoindoline in 65 ml of hot THF is added. The reaction mixture is stirred at reflux for 2 hours. After cooling to 0°C, 60 ml of methanolic hydrochloric acid is added dropwise. The solution is stirred at reflux for 4.5 hours and the solvents are removed under vacuum. The resulting orange oil is stirred with methanolic HCl under a nitrogen atmosphere for 48 hours. The methanol is evaporated, and the residue is washed several times with methanol. The residue is dried under vacuum to yield 5.3 grams of 2-benzyl-3a,4,5,9b-tetrahydro-6,7-dimethoxybenzo[e]isoindoline hydrochloride as an orange glass.

Example 6

3a,4,5,9b-tetrahydro-6,7-dimethoxybenzo[e]isoindoline hydrochloride

5.3 grams of 2-benzyl-3a,4,5,9b-tetrahydro-6,7-dimethoxybenzo[e]isoindoline hydrochloride in 100 ml of ethanol is hydrogenated under 3 atmospheres of hydrogen at 60°C in the presence of 1 gram of 5% palladium-on-carbon catalyst. The catalyst is removed by filtration, the solvent is evaporated, and the residue dissolved in aqueous sodium hydroxide. The residue is then extracted with three 50-ml portions of methylene dichloride and the combined extracts are washed with water and then with a saturated aqueous sodium chloride solution. The residue is then dried over magnesium sulfate, filtered, and the solvents are removed under vacuum to yield 3.0 grams of 3a,4,5,9b-tetrahydro-6,7-dimethoxybenzo[e]isoindoline as an oily orange solid. A portion of the orange solid is converted to the hydrochloride salt by treatment with diethyl ether and hydrochloric acid. Recrystallization from ethanol/diethyl ether yields 0.25 grams of 3a,4,5,9b-tetrahydro-6,7-dimethoxybenzo[e]isoindoline hydrochloride as a white solid, m.p. 141—143°C.

Example 7

1,3-dioxo-2-methyl-3a,4,5,9b-tetrahydro-6,7-dimethoxybenzo[e]isoindoline

A slurry of 2.97 grams of 1,3-dioxo-3a,4,5,9b-tetrahydro-6,7-dimethoxybenzo[e]isoindoline in 50 ml of dry DMF is stirred under a nitrogen atmosphere at room temperature as a suspension of 0.6 grams of sodium hydride (50% in oil, washed with tetrane) and 6 ml of dry DMF is added in portions over 0.5 hours. The reaction mixture is stirred for 1.5 hours, and then 1.2 ml of dimethyl sulfate is added dropwise to obtain a clear solution which is stirred overnight at room temperature. The solution is evaporated to dryness to yield a yellow solid. Water is added and the solution is extracted with three 50-ml portions of methylene dichloride. The extracts are combined and washed with two 100-ml portions of water, and then one 100-ml portion of saturated aqueous sodium chloride. The solution is dried over sodium sulfate, filtered, and the solvent is evaporated to yield 3.0 grams of 1,3-dioxo-2-methyl-3a,4,5,9b-tetrahydro-6,7-dimethoxybenzo[e]isoindoline as a yellow solid, m.p. 131—139°C.

Example 8

2-methyl-3a,4,5,9b-tetrahydro-6,7-dimethoxybenzo[e]isoindoline hydrochloride

The procedure of Example 5 is repeated using 1,3-dioxo-2-methyl-3a,4,5,9b-tetrahydro-6,7-dimethoxybenzo[e]isoindoline as the starting material to obtain 2-methyl-3a,4,5,9b-tetrahydro-6,7-dimethoxybenzo[e]isoindoline hydrochloride as a white solid, m.p. 165—168°C.

Example 9

2-methyl-3a,4,5,9b-tetrahydro-6,7-dihydroxybenzo[e]isoindoline hydrobromide

A solution of 0.47 grams of 2-methyl-3a,4,5,9b-tetrahydro-6,7-dimethoxybenzo[e]isoindoline hydrochloride in 4.7 ml of dry methylene dichloride is stirred under a nitrogen atmosphere at −78°C as a solution of 0.64 ml of $BBr_3$ and 2.4 ml of methylene dichloride is added dropwise. The reaction mixture is stirred for 2 hours at −78°C and then for an additional 2.25 hours at 0°C. The reaction is quenched at −78°C with the dropwise addition of 12 ml of methanol. The reaction mixture is stirred for 0.5 hours at −78°C, and then overnight at room temperature. The resulting precipitate is filtered, washed with diethyl ether, and then dried under vacuum to obtain 0.35 grams of 2-methyl-3a,4,5,9b-tetrahydro-6,7-dihydroxybenzo[e]isoindoline hydrobromide as a tan solid, m.p. 224—226°C.

Example 10

3a,4,5,9b-tetrahydro-6,7-dimethoxybenzo[e]isoindoline

The procedure of Example 5 is repeated using 1,3-dioxo-3a,4,5,9b-tetrahydro-6,7-dimethoxybenzo[e]isoindoline as the starting material to obtain 3a,4,5,9b-tetrahydro-6,7-dimethoxybenzo[e]isoindoline as an oil identical to that obtained in Example 6.

Example 11

3a,4,5,9b-tetrahydro-6,7-dihydroxybenzo[e]isoindoline hydrobromide

The procedure of Example 9 is repeated using 3a,4,5,9b-tetrahydro-6,7-dimethoxybenzo[e]isoindoline as the starting material to obtain 3a,4,5,9b-tetrahydro-6,7-dihydroxybenzo[e]isoindoline hydrobromide as a white solid, m.p. greater than 250°C.

Example 12

2-n-propyl-3a,4,5,9b-tetrahydro-6,7-dimethoxybenzo[e]isoindoline hydrochloride

A slurry of 0.68 grams of sodium hydride (50% in oil, washed with tetrane) and 68 ml of dry DMF is stirred under a nitrogen atmosphere at room temperature as a solution of 3.0 grams of 3a,4,5,9b-tetrahydro-6,7-dimethoxybenzo[e]isoindoline and 20 ml of dry DMF is added dropwise. The reaction mixture is stirred for 1.5 hours at room temperature, and then 1.3 ml of 1-bromo propane is added dropwise. The reaction mixture is stirred 1 hour at room temperature, for 2 hours at 35—45°C, and then overnight at 30°C. The reaction mixture is evaporated to dryness. Water is added to the residue and the aqueous layer is extracted with three 75-ml portions of methylene dichloride. The combined extracts are washed with water, saturated aqueous sodium chloride, and then dried over magnesium sulfate. The solution is filtered and evaporated to yield 2.9 grams of an orange oil. The oil is converted to the hydrochloride salt by treatment with ethanol and ethereal hydrochloric acid, and then crystallized to obtain 2.0 grams of 2-n-propyl-3a,4,5,9b-tetrahydro-6,7-dimethoxybenzo[e]isoindoline hydrochloride as a gray glass.

Example 13

2-n-propyl-3a,4,5,9b-tetrahydro-6,7-dihydroxybenzo[e]isoindoline hydrobromide

The procedure of Example 9 is repeated using 2-n-propyl-3a,4,5,9b-tetrahydro-6,7-dimethoxybenzo[e]isoindoline hydrochloride as the starting material to obtain 2-n-propyl-3a,4,5,9b-tetrahydro-6,7-dihydroxybenzo[e]isoindoline hydrobromide as a gray powder, m.p. 228—230°C.

Example 14

(2'*S*)-2(1',4'-benzodioxan-2'-methyl)-3a,4,5,9b-tetrahydro-6,7-dimethoxybenzo[e]isoindoline hydrochloride

A solution of 210 ml of 3a,4,5,9b-tetrahydro-6,7-dimethoxybenzo[e]isoindoline, 0.9 ml of $CH_3CN$ and 0.6 ml of diisopropyl ethyl amine is stirred under a nitrogen atmosphere at room temperature. To the reaction mixture is added 0.4 grams of (2*R*)-2-tosyloxymethyl-1,4-benzodioxan and the reaction mixture is stirred overnight at reflux. The reaction mixture is cooled to room temperature and the solvents are evaporated. The residue is shaken with saturated aqueous sodium bicarbonate solution and ether. The layers are separated, and the aqueous layer is extracted with two 100-ml portions of ether. The combined organic layers are washed with saturated aqueous sodium chloride, dried over magnesium sulfate, filtered and evaporated to yield an orange oil. The oil is converted to hydrochloride salt with methanol and ethereal hydrochloric acid to yield 0.26 grams of (2'*S*)-2(1',4'-benzodioxan-2'-methyl)-3a,4,5,9b-tetrahydro-6,7-dimethoxybenzo[e]isoindoline hydrochloride as a gray solid, m.p. 202—205°C.

Example 15

1,2-dicyano-6,7-dimethoxy-1,2,3,4-tetrahydronaphthalene

The procedure of Example 2 is repeated using 1-cyano-6,7-dimethoxy-3,4-dihydronaphthalene as the starting material to obtain 1,2-dicyano-6,7-dimethoxy-1,2,3,4-tetrahydronaphthalene, m.p. 155—163°C.

Example 16

1,3-dioxo-3a,4,5,9b-tetrahydro-7,8-dimethoxybenzo[e]isoindoline

A solution of 2.89 grams of 1,2-dicyano-6,7-dimethoxy-1,2,3,4-tetrahydronaphthalene in 29 ml of dry methylene dichloride is stirred at 0°C as hydrogen bromide gas is passed through the solution for 1.5 hours. To the solution is added 35 ml of ether, and the resulting solid is filtered, dried under vacuum, and then boiled in an open flask on a steam bath with a solution of 29 ml of water and 14.5 ml of DMF for 2 hours. The resulting black solution is stored at 0°C for 2 days. The solid phase is removed by filtration, washed with water, and dried under vacuum to obtain 1.7 grams of 1,3-dioxo-3a,4,5,9b-tetrahydro-7,8-dimethoxybenzo[e]iso-indoline as a tan solid, m.p. 200—208°C.

Example 17

3a,4,5,9b-tetrahydro-7,8-dimethoxybenzo[e]isoindoline hydrochloride

The procedure of Example 5 is repeated using 1,3-dioxo-3a,4,5,9b-tetrahydro-7,8-dimethoxybenzo[e]isoindoline as the starting material to obtain 3a,4,5,9b-tetrahydro-7,8-dimethoxybenzo[e]isoindoline hydrochloride, m.p. 245—247°C.

Example 18

3a,4,5,9b-tetrahydro-7,8-dihydroxybenzo[e]isoindoline hydrobromide

The procedure of Example 9 is repeated using 3a,4,5,9b-tetrahydro-7,8-dimethoxybenzo[e]isoindoline hydrochloride as the starting material to obtain 3a,4,5,9b-tetrahydro-7,8-dihydroxybenzo[e]isoindoline hydrobromide.

Example 19

2-(2'-(*o*-methoxyphenethyl))-3a,4,5,9b-tetrahydro-7,8-dimethoxybenzo[e]isoindoline hydrochloride

The procedure of Example 5 is repeated using 2-(*o*-methoxyphenylacetyl)-3a,4,5,9b-tetrahydro-7,8-dimethoxybenzo[e]isoindoline as the starting material to obtain 2-(2'-(*o*-methoxyphenethyl-3a,4,5,9b-tetrahydro-7,8-dimethoxybenzo[e]isoindoline hydrochloride.

Example 20

2-(2'-(*o*-hydroxyphenethyl))-3a,4,5,9b-tetrahydro-7,8-dihydroxybenzo[e]isoindoline hydrobromide

The procedure of Example 9 is repeated using 2-(2'-(*o*-methoxyphenethyl))-3a,4,5,9b-tetrahydro-7,8-dimethoxybenzo[e]isoindoline hydrochloride as the starting material to obtain 2-(2'-(*o*-hydroxyphenethyl))-3a,4,5,9b-tetrahydro-7,8-dihydroxybenzo[e]isoindoline hydrobromide.

Example 21

2-(2'-phenoxyethyl)-3a,4,5,9b-tetrahydro-7,8-dimethoxybenzo[e]isoindoline hydrochloride

The procedure of Example 5 is repeated using 2-phenoxyacetyl-3a,4,5,9b-tetrahydro-7,8-dimethoxybenzo[e]isoindoline as the starting material to obtain 2-(2'-phenoxyethyl)-3a,4,5,9b-tetrahydro-7,8-dimethoxybenzo[e]isoindoline hydrochloride.

Example 22

2-(3'-(*o*-methoxyphenyl)-propyl)-3a,4,5,9b-tetrahydro-7,8-dimethoxybenzo[e]isoindoline hydrochloride

The procedure of Example 5 is repeated using 2-(3'-(o-methoxyphenyl)propionyl-3a,4,5,9b-tetrahydro-7,8-dimethoxybenzo[e]isoindoline as the starting material to obtain 2-(3'-(*o*-methoxyphenyl)-propyl)-3a,4,5,9b-tetrahydro-7,8-dimethoxybenzo[e]isoindoline hydrochloride.

Example 23

2-(3'-(*o*-hydroxyphenyl)-propyl)-3a,4,5,9b-tetrahydro-7,8-dihydroxybenzo[e]isoindoline hydrobromide

The procedure of Example 9 is repeated using 2-(3'-(*o*-methoxyphenyl)-propyl)-3a,4,5,9b-tetrahydro-7,8-dimethoxybenzo[e]isoindoline hydrochloride as the starting material to obtain 2-(3'-(*o*-hydroxyphenyl)propyl)-3a,4,5,9b-tetrahydro-7,8-dihydroxybenzo[e]isoindoline hydrobromide.

Example 24

(2'*S*)-2-(1',4'-benzodioxan-2'-methyl)-3a,4,5,9b-tetrahydro-7,8-dimethoxybenzo[e]isoindoline hydrochloride

The procedure of Example 14 is repeated using 3a,4,5,9b-tetrahydro-7,8-dimethoxybenzo[e]isoindoline hydrochloride as the starting material to obtain (2'*S*)-2-(1',4'-benzodioxan-2'-methyl)-3a,4,5,9b-tetrahydro-7,8-dimethoxybenzo[e]isoindoline hydrochloride.

The therapeutic activity of the compounds can be demonstrated in vivo by their ability to decrease arterial blood pressure and/or heart rate in the spontaneously hypertensive rat as follows. A group of Okamoto rats, which develop hypertension spontaneously when reaching young adulthood, are deprived of food for a period of 16 hours and are placed in semi-restraining wire mesh cylinders maintained at a constant temperature of 36°C. An occluding cuff, operatively connected to a programmed sphygmomanometer, is placed over the tail of each rat of the group and retained near the tail base. The pressure of each cuff is automatically, cyclically increased within the range of from 0 to 250 mm Hg at the rate of 10 mm Hg/sec, the total inflation and deflation time of each cycle being 50 seconds, with a 10 second rest period between cycles. A photocell is placed distal to the cuff to detect pulses resulting from the forward motion of blood flow with each heartbeat of the rat. As the pressure in the cuff increases, measurable pulses disappear at the point where the cuff pressure equals the arterial blood pressure. Measurable pulses reappear during deflation at approximately the same pressure, and aterial blood pressure is thereby established by cuff pressure at the point of pulse appearance. The heart rate is determined from the arterial pulse wave. A 100 mg/kg dose of a test compound of formula I is administered orally to each rat of the test group, and five interference-free signals are recorded on a Model 7 Grass polygraph for each rat at various measurement periods following administration. By following the foregoing procedure, the tested preferred compounds of the invention are shown to decrease the arterial blood pressure and/or heart rate of rats of the group.

The compounds of the invention can be administered in any effective pharmaceutically acceptable form to warm blooded animals, e.g., in oral, parenteral or infusable dosage forms, or as a buccal or nasal spray. Suitable parenteral routes of administration include, for example, intramuscular, intravenous, intraperitoneal or subcutaneous administration of the compounds.

In addition to the active compounds, compositions according to this invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be sterilized, for example, by filtration through a bacteria-retaining filter, or by incorporating sterilizing agents into the compositions. They can also be manufactured in the form of sterile solid compositions which can be dissolved in sterile water, or other sterile injectable medium, immediately before use.

Solid dosage forms for oral administration include capsules, tablets, pills, powders and granules. In such solid dosage forms, the active compound may be admixed with at least one inert diluent such as sucrose, lactose or starch. Such dosage forms may also comprise, as is normal practice, additional substances other than inert diluents, e.g., lubricating agents such as magnesium stearate. In the case of

capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixirs containing inert diluents commonly used in.the art, such as water. Besides such inert diluents, compositions may also comprise adjuvants, such as wetting agents, emulsifying and suspending agents, and sweetening, flavoring and perfuming agents.

Actual dosage levels of active ingredient in the compositions of the invention may be varied so as to obtain an amount of active ingredient effective to obtain a desired therapeutic response for a particular composition and method of administration. The selected dosage level therefore depends upon the desired therapeutic effect, on the route of administration, on the desired duration of treatment and other factors. Generally, dosage levels of about 0.1 to about 200, more preferably about 0.5 to about 150 and most preferably about 1 to about 125 mg of active ingredient per kg of body weight per day are administered orally to a mammalian patient suffering from hypertension. If desired, the daily dose may be divided into multiple doses for administration, e.g., two to four separate doses per day.

**Claims for the Contracting States: BE CH DE FR GB IT LI LU NL SE**

1. A compound of the formula

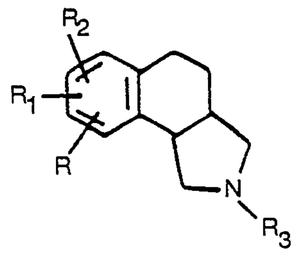

wherein R, $R_1$, and $R_2$ are independently selected from hydrogen, hydroxy, alkoxy of 1 to 3 carbon atoms, or R and $R_1$, or $R_1$ and $R_2$ can be taken together to form a methylenedioxy or ethylenedioxy bridge; with the proviso that at least one of R, $R_1$ or $R_2$ must be other than hydrogen; and $R_3$ is hydrogen; alkyl of 1 to 4 carbon atoms; a substituent of the formula

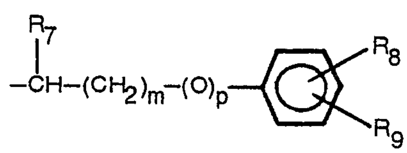

wherein m is 0, 1 or 2, p is 0 or 1, $R_7$ is hydrogen or alkyl of 1 to 4 carbon atoms and $R_8$ and $R_9$ are independently selected from hydrogen, hydroxy, methoxy, alkyl of 1 to 4 carbon atoms, or halo, or $R_8$ and $R_9$ can be taken together to form a methylenedioxy or ethylenedioxy bridge; or 1,4-benzodioxan of the formula

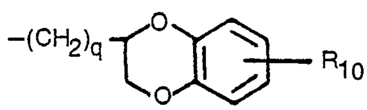

wherein q is 1, 2 or 3, and $R_{10}$ is hydrogen, methoxy, or halo; and the pharmaceutically acceptable salts thereof.

2. A compound of Claim 1 wherein one of R, $R_1$, and $R_2$ is hydrogen and the remaining two of R, $R_1$ and $R_2$ are hydroxy.

3. A compound of Claim 1 wherein one of R, $R_1$ and $R_2$ is hydrogen and the remaining two of R, $R_1$ and $R_2$ are methoxy.

4. A compound of Claim 1 wherein R and $R_1$ are taken together to form methylenedioxy.

5. A compound of Claim 1 wherein $R_1$ and $R_2$ are taken together to form methylene dioxy.

6. A compound of Claims 1, 2, 3, 4, or 5 wherein $R_3$ is hydrogen.

7. A compound of Claims 1, 2, 3, 4, or 5 wherein $R_3$ is isopropyl.

8. A compound of Claims 1, 2, 3, 4, or 5 wherein $R_3$ is aryloxyalkyl of the formula

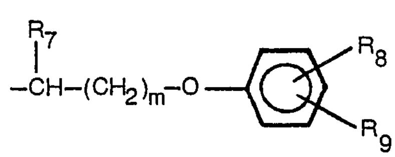

wherein m, $R_7$, $R_8$ and $R_9$ are as defined in Claim 1.

9. A compound of Claims 1, 2, 3, 4, or 5 wherein $R_3$ is arylalkyl of the formula

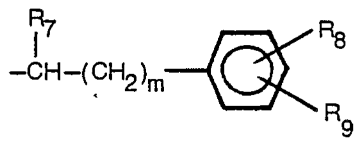

wherein m, $R_7$, $R_8$ and $R_9$ are as defined in Claim 1.

10. A compound of Claims 1, 2, 3, 4, and 5 wherein $R_3$ is methyl.

11. A compound of Claims 1, 2, 3, 4, or 5 wherein $R_3$ is a benzodioxane of the formula

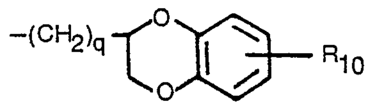

wherein q and $R_{10}$ are as defined in Claim 1.

12. A composition in pharmaceutical dosage form comprising the compound of claim 1 and the pharmaceutically acceptable salts thereof, and a pharmaceutically acceptable carrier or diluent.

**Claims for the Contracting State: AT**

1. A process for preparing a compound of the formula

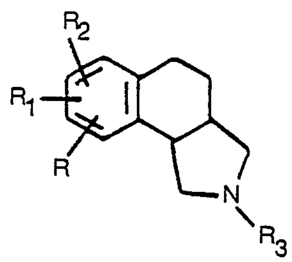

(I)

wherein R, $R_1$, and $R_2$ are independently selected from hydrogen, hydroxy, alkoxy of 1 to 3 carbon atoms, or R and $R_1$, or $R_1$ and $R_2$ can be taken together to form a methylenedioxy or ethylenedioxy bridge; with the proviso that at least one of R, $R_1$ or $R_2$ must be other than hydrogen; and $R_3$ is hydrogen; alkyl of 1 to 4 carbon atoms; a substituent of the formula

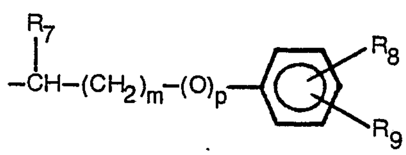

wherein m is 0, 1 or 2, p is 0 or 1, $R_7$ is hydrogen or alkyl of 1 to 4 carbon atoms and $R_8$ and $R_9$ are independently selected from hydrogen, hydroxy, methoxy, alkyl of 1 to 4 carbon atoms, or halo, or $R_8$ and $R_9$ can be taken together to form a methylenedioxy or ethylenedioxy bridge; or 1,4-benzodioxan of the formula

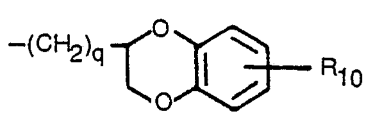

wherein q is 1, 2 or 3, and $R_{10}$ is hydrogen, methoxy, or halo; and pharmaceutically acceptable salts thereof, comprising

(i) reducing a compound (4) wherein R', $R_1'$ and $R_2'$ are independently selected from hydrogen and alkoxy of 1 to 3 carbon atoms, at least one of R', $R_1'$ and $R_2'$ being other than hydrogen, to obtain a 3a,4,5,9b-tetrahydro-benzo-[e]isoindoline (I) wherein $R_3$ is hydrogen, and R, $R_1$ and $R_2$ are not hydroxy, .

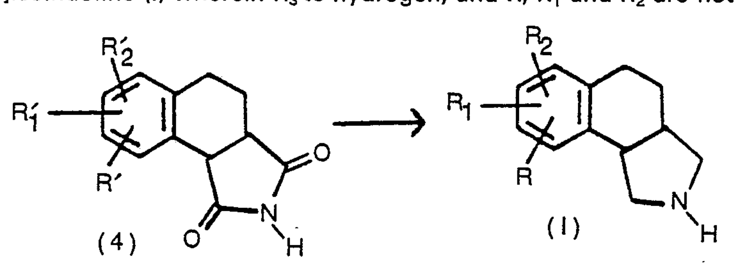

(4) (I)

(ii) reacting (I) with an alkylating agent $R_3X$ to obtain a compound (I) wherein $R_3$ is different from hydrogen or reacting (I) with an acylating agent selected from $R_3$—COX and $R_3$—$SO_2X$ followed by reducing the resulting amide, to obtain a compound (I) wherein $R_3$ is different from hydrogen,

(iii) and optionally dealkylating compound (I) obtained in (ii) to obtain a compound (I) wherein R, and/or $R_1$ and/or $R_2$ is hydroxy.

2. The process as described in Claim 1 further comprising one or more of the preliminary steps of

(a) reacting a substituted tetralone (1) with trimethylsilylcyanide and then with HCl to obtain a 1-cyano-3,4-dihydronaphthalene (2)

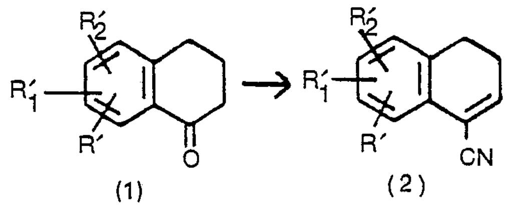

(1) (2)

(b) reacting (2) with a cyanide ion to obtain an 1,2-dicyano-1,2,3,4-tetrahydronaphthalene (3)

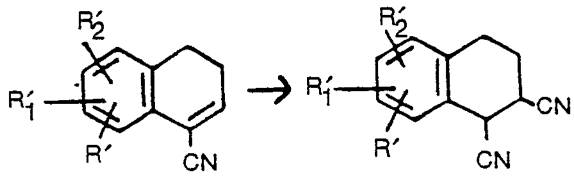

(2) (3)

(c) and treating compound (3) either with sulphuric acid or with hydrogen bromide gas and DMF to yield said compound (4).

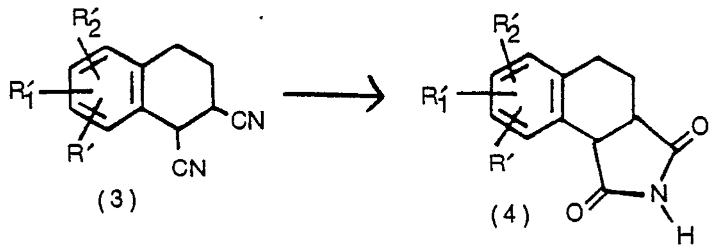

(3) (4)

3. A process of claim 1 for preparing a compound I wherein one of R, $R_1$, and $R_2$ is hydrogen and the remaining two of R, $R_1$ and $R_2$ are hydroxy.

4. A process of Claim 1 for preparing a compound I wherein one of R, $R_1$ and $R_2$ is hydrogen and the remaining two of R, $R_1$ and $R_2$ are methoxy.

5. A process of Claim 1 for preparing a compound I wherein R and $R_1$ are taken together to form methylendioxy.

6. A process of Claim 1 for preparing a compound I wherein $R_1$ and $R_2$ are taken together to form methylene dioxy.

7. A process of claims 1, 2, 3, 4, 5 or 6 for preparing a compound I wherein $R_3$ is hydrogen.

8. A process of Claims 1, 2, 3, 4, 5 or 6 for preparing a compound I wherein $R_3$ is isopropyl.

9. A process of Claims 1, 2, 3, 4, 5 or 6 for preparing a compound I wherein $R_3$ is aryloxyalkyl of the formula

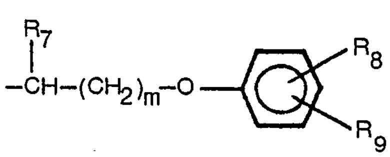

wherein m, $R_7$, $R_8$ and $R_9$ are as defined in Claim 1.

10. A process of Claims 1, 2, 3, 4, 5 or 6 for preparing a compound I wherein $R_3$ is arylalkyl of the formula

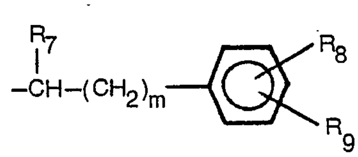

wherein m, $R_7$, $R_8$, and $R_9$ are as defined in Claim 1, and wherein $R_4$ is hydrogen.

11. A process of Claims 1, 2, 3, 4, 5 or 6 for preparing a compound I wherein $R_3$ is methyl.

12. A process of Claims 1, 2, 3, 4, 5 or 6 for preparing a compound I wherein $R_3$ is a benzodioxane of the formula

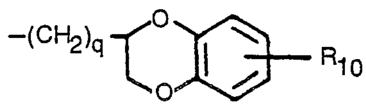

wherein q and $R_{10}$ are as defined in Claim 1.

13. A process for preparing a composition in pharmaceutical dosage form comprising mixing a compound prepared according to the process of Claim 1 or a pharmaceutically acceptable salts thereof, with a pharmaceutically acceptable carrier or diluent.

**Patentansprüche für die Vertragsstaaten: BE CH DE FR GB IT LI LU NL SE**

1. Eine Verbindung der Formel

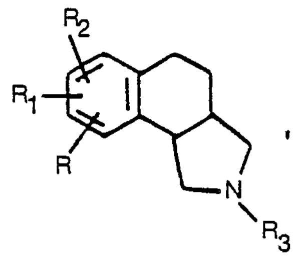

,

worin R, $R_1$ und $R_2$ unabhängig voneinander aus Wasserstoff, Hydroxy und Alkoxy mit 1 bis 3 Kohlenstoffatomen ausgewählt sind, oder R und $R_1$, oder $R_1$ und $R_2$ zusammengenommen eine Methylendioxy- oder Ethylendioxybrücke bilden können; mit der Maßgabe, daß wenigstens einer der Reste R, $R_1$ oder $R_2$ von Wasserstoff verschieden sein muß; und $R_3$ Wasserstoff; Alkyl mit 1 bis 4 Kohlenstoffatomen; ein Substituent der Formel

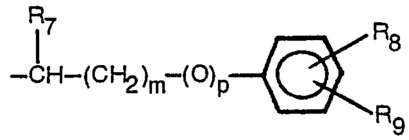

,

worin m 0, 1 oder 2 ist, p 0 oder 1 ist, $R_7$ Wasserstoff oder Alkyl mit 1 bis 4 Kohlenstoffatomen ist, und $R_8$ und $R_9$ unabhängig voneinander aus Wasserstoff, Hydroxy, Methoxy, Alkyl mit 1 bis 4 Kohlenstoffatomen oder Halogen ausgewählt sind, oder $R_8$ und $R_9$ zusammengenommen eine Methylendioxy- oder Ethylendioxybrücke bilden können; oder 1,4-Benzodioxan der Formel

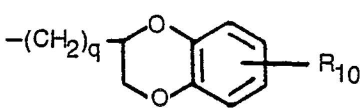

ist, worin q 1, 2 oder 3 ist, und $R_{10}$ Wasserstoff, Methoxy oder Halogen ist; und die pharmazeutisch annehmbaren Salze davon.

2. Eine Verbindung nach Anspruch 1, wobei einer der Reste R, $R_1$ und $R_2$ Wasserstoff ist, und die übrigen zwei der Reste R, $R_1$ und $R_2$ Hydroxy sind.

3. Eine Verbindung nach Anspruch 1, wobei einer der Reste R, $R_1$ und $R_2$ Wasserstoff ist, und die übrigen zwei der Reste R, $R_1$ und $R_2$ Methoxy sind.

4. Eine Verbindung nach Anspruch 1, wobei R und $R_1$ zusammengenommen Methylendioxy bilden.

5. Eine Verbindung nach Anspruch 1, wobei $R_1$ und $R_2$ zusammengenommen Methylendioxy bilden.

6. Eine Verbindung nach Anspruch 1, 2, 3, 4 oder 5, wobei $R_3$ Wasserstoff ist.

7. Eine Verbindung nach Anspruch 1, 2, 3, 4 oder 5, wobei $R_3$ Isopropyl ist.

8. Eine Verbindung nach Anspruch 1, 2, 3, 4 oder 5, wobei $R_3$ Aryloxyalkyl der Formel

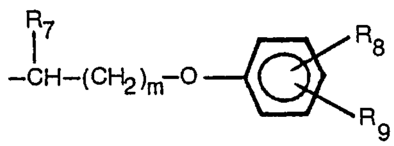

ist, wobei m, $R_7$, $R_8$ und $R_9$ wie in Anspruch 1 definiert sind.

9. Eine Verbindung nach Anspruch 1, 2, 3, 4 oder 5, wobei $R_3$ Arylalkyl der Formel

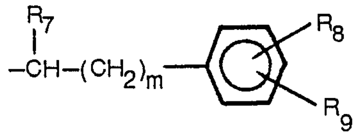

ist, worin m, $R_7$, $R_8$ und $R_9$ wie in Anspruch 1 definiert sind.

10. Eine Verbindung nach einem der Ansprüche 1, 2, 3, 4 und 5, wobei $R_3$ Methyl ist.

11. Eine Verbindung nach Anspruch 1, 2, 3, 4 oder 5, wobei $R_3$ ein Benzodioxan der Formel

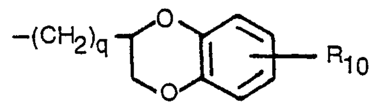

ist, worin q und $R_{10}$ wie in Anspruch 1 definiert sind.

12. Eine Zusammensetzung in pharmazeurischer Dosierungsform, enthaltend eine Verbindung nach Anspruch 1, oder ein pharmazeutisch annehmbares Salz davon, und einen pharmazeutisch annehmbaren Träger oder ein pharmazeutisch annehmbares Verdünnungsmittel.

**Patentansprüche für den Vertragsstaat: AT**

1. Ein Verfahren zur Herstellung einer Verbindung der Formel

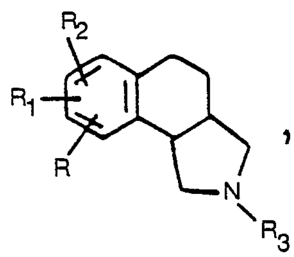

, (I)

worin R, $R_1$ und $R_2$ unabhängig voneinander aus Wasserstoff, Hydroxy und Alkoxy mit 1 bis 3 Kohlenstoffatomen ausgewählt sind, oder R und $R_1$, oder $R_1$ und $R_2$ zusammengenommen eine Methylendioxy- oder Ethylendioxybrücke bilden können; mit der Maßgabe, daß wenigstens einer der Reste R, $R_1$ oder $R_2$ von Wasserstoff verschieden sein muß; und $R_3$ Wasserstoff; Alkyl mit 1 bis 4 Kohlenstoffatomen; ein Substituent der Formel

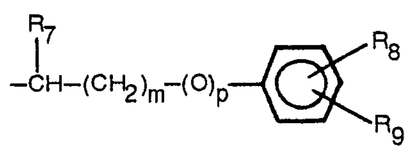

,

worin m 0, 1 oder 2 ist, p 0 oder 1 ist, $R_7$ Wasserstoff oder Alkyl mit 1 bis 4 Kohlenstoffatomen ist, und $R_8$ und $R_9$ unabhängig voneinander aus Wasserstoff, Hydroxy, Methoxy, Alkyl mit 1 bis 4 Kohlenstoffatomen oder Halogen ausgewählt sind, oder $R_8$ und $R_9$ zusammengenommen eine Methylendioxy- oder Ethylendioxybrücke bilden können; oder 1,4-Benzodioxan der Formel

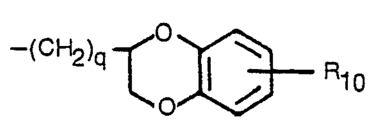

ist, worin q 1, 2 oder 3 ist, und $R_{10}$ Wasserstoff, Methoxy oder Halogen ist; und die pharmazeutisch annehmbaren Salze davon, umfassend

(i) das Reduzieren einer Verbindung (4), worin R′, $R_1'$ und $R_2'$ unabhängig voneinander aus Wasserstoff und Alkoxy mit 1 bis 3 Kohlenstoffatomen ausgewählt sind, und wenigstens einer der Reste R′, $R_1'$ und $R_2'$ von Wasserstoff verschieden ist, zur Gewinnung eines 3a,4,5,9b-Tetrahydro-benzo-[e]-isoindolins (I), worin $R_3$ Wasserstoff ist, und R, $R_1$ und $R_2$ nicht Hydroxy sind,

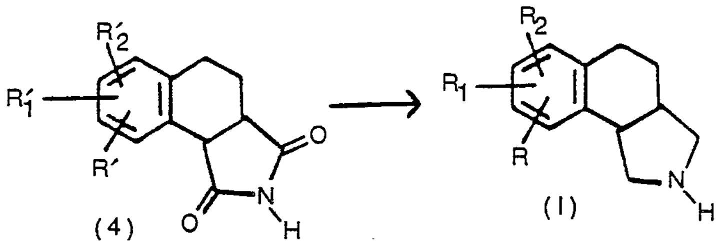

(4) (I)

(ii) das Umsetzen von (I) mit einem Alkylierungsmittel $R_3X$ zur Gewinnung einer Verbindung (I), worin $R_3$ von Wasserstoff verschieden ist, oder das Umsetzen von (I) mit einem Acylierungsmittel, das aus $R_3$—COX und $R_3$—$SO_2X$ ausgewählt ist, gefolgt vom Reduzieren des entstandenen Amids, zur Gewinnung einer Verbindung (I), worin $R_3$ von Wasserstoff verschieden ist, und

(iii) gegebenenfalls das Entalkylieren der gemäß (ii) erhaltenen Verbindung (I) zur Gewinnung einer Verbindung (I), worin R und/oder $R_1$ und/oder $R_2$ Hydroxy ist bzw. sind.

2. Das wie in Anspruch 1 beschriebene Verfahren, welches weiterhin eine oder mehrere der nachstehend angegebenen Vorstufen umfaßt:

(a) das Umsetzen eines substituierten Tetralons (1) mit Trimethylsilylcyanid und dann mit HCl zur Gewinnung eines 1-Cyano-3,4-dihydronaphthalins (2)

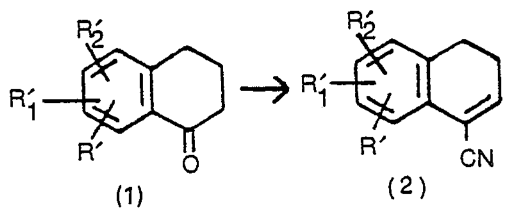

(1) (2)

(b) das Umsetzen von (2) mit einem Cyanidion zur Gewinnung eines 1,2-Dicyano-1,2,3,4-tetrahydronaphthalins (3)

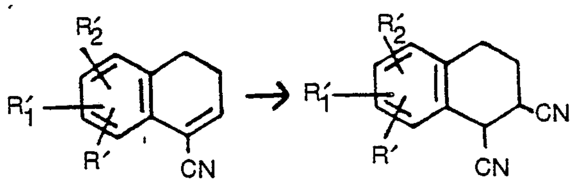

(2) (3)

und

(c) das Behandeln von Verbindung (3) entweder mit Schwefelsäure oder mit Bromwasserstoffgas und DMF zur Gewinnung der genannten Verbindung (4)

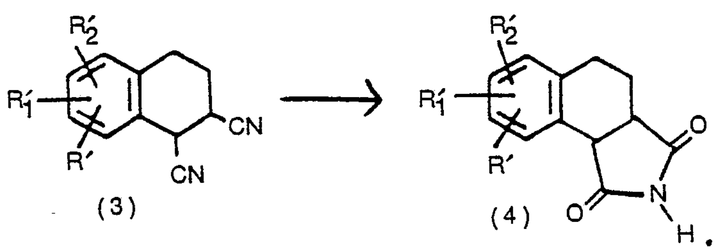

(3) (4)

3. Ein Verfahren nach Anspruch 1 zur Herstellung einer Verbindung I, worin einer der Reste R, $R_1$ und $R_2$ Wasserstoff ist, und die übrigen zwei der Reste R, $R_1$ und $R_2$ Hydroxy sind.

4. Ein Verfahren nach Anspruch 1 zur Herstellung einer Verbindung I, worin einer der Reste R, $R_1$ und $R_2$ Wasserstoff ist, und die übrigen zwei der Reste R, $R_1$ und $R_2$ Methoxy sind.

5. Ein Verfahren nach Anspruch 1 zur Herstellung einer Verbindung I, worin R und $R_1$ zusammengenommen Methylendioxy bilden.

6. Ein Verfahren nach Anspruch 1 zur Herstellung einer Verbindung I, worin $R_1$ und $R_2$ zusammengenommen Methylendioxy bilden.

7. Ein Verfahren nach Anspruch 1, 2, 3, 4, 5 oder 6 zur Herstellung einer Verbindung I, worin $R_3$ Wasserstoff ist.

8. Ein Verfahren nach Anspruch 1, 2, 3, 4, 5 oder 6 zur Herstellung einer Verbindung I, worin $R_3$ Isopropyl ist.

9. Ein Verfahren nach einem der Ansprüche 1, 2, 3, 4, 5 oder 6 zur Herstellung einer Verbindung I, worin $R_3$ Aryloxyalkyl der Formel

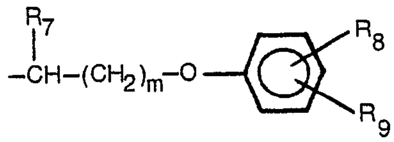

ist, wobei m, $R_7$, $R_8$ und $R_9$ wie in Anspruch 1 definiert sind.

10. Ein Verfahren nach Anspruch 1, 2, 3, 4, 5 oder 6 zur Herstellung einer Verbindung I, worin $R_3$ Arylalkyl der Formel

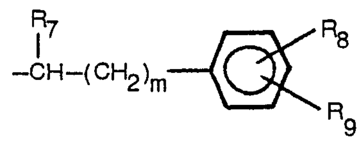

ist, wobei m, $R_7$, $R_8$ und $R_9$ wie in Anspruch 1 definiert sind, und worin $R_4$ Wasserstoff ist.

11. Ein Verfahren nach Anspruch 1, 2, 3, 4, 5 oder 6 zur Herstellung einer Verbindung I, worin $R_3$ Methyl ist.

12. Ein Verfahren nach Anspruch 1, 2, 3, 4, 5 oder 6 zur Herstellung einer Verbindung I, worin $R_3$ ein Benzodioxan der Formel

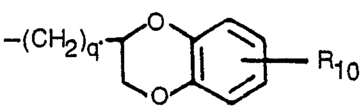

ist, wobei q und $R_{10}$ wie in Anspruch 1 definiert sind.

13. Ein Verfahren zur Herstellung einer Zusammensetzung in pharmazeutischer Dosierungsform, umfassend das Vermischen einer nach dem Verfahren des Anspruchs 1 hergestellten Verbindung, oder eines pharmazeutisch annehmbaren Salzes davon, mit einem pharmazeutisch annehmbaren Träger oder einem pharmazeutisch annehmbaren Verdünnungsmittel.

**Revendications pour les Etats contractants: BE CH DE FR GB IT LI LU NL SE**

1. Un composé de formule

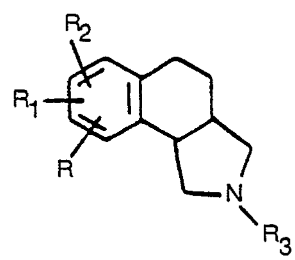

dans laquelle R, $R_1$ et $R_2$ sont choisis indépendamment parmi l'hydrogène, l'hydroxy, les groupes alcoxy de 1 à 3 atomes de carbone, où R et $R_1$, ou $R_1$ et $R_2$ peuvent être pris ensemble pour former un pont méthylènedioxy ou éthylènedioxy; à la condition qu'au moins l'un des R, $R_1$ ou $R_2$ doit être autre que l'hydrogène; et $R_3$ est l'hydrogène; un alkyle à 1 à 4 atomes de carbone; un substituant de formule

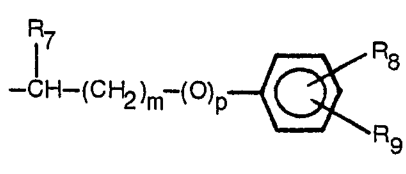

dans laquelle m est 0, 1 ou 2, p est 0 ou 1, $R_7$ est l'hydrogène ou un alkyle ayant 1 à 4 atomes de carbone et $R_8$ et $R_9$ sont choisis indépendamment parmi l'hydrogène, l'hydroxy, le groupe méthoxy, les groupes alkyles de 1 à 4 atomes de carbone, ou halo, ou $R_8$ et $R_9$ peuvent être pris ensemble pour former un pont méthylènedioxy ou éthylènedioxy; ou bien 1,4-benzodioxanne de formule

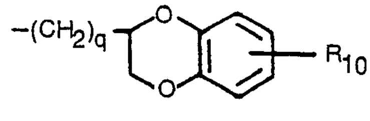

dans laquelle q est égal à 1, 2 ou 3, et $R_{10}$ est l'hydrogène, un groupe méthoxy, ou halo; ainsi que les sels pharmaceutiquement acceptables de ces composés.

2. Un composé selon la revendication 1, selon lequel l'un des R, $R_1$ et $R_2$ est l'hydrogène et les deux autres des R, $R_1$ et $R_2$ sont des groupes hydroxy.

3. Un composé selon la revendication 1, selon lequel l'un des R, $R_1$ et $R_2$ est l'hydrogène et les deux autres des R, $R_1$ et $R_2$ sont des groupes méthoxy.

4. Un composé selon la revendication 1, selon lequel R et $R_1$ sont pris ensemble pour former un groupe méthylènedioxy.

5. Un composé selon la revendication 1, selon lequel $R_1$ et $R_2$ sont pris ensemble pour former un groupe méthylènedioxy.

6. Un composé selon la revendication 1, 2, 3, 4 ou 5, selon lequel $R_3$ est l'hydrogène.

7. Un composé selon la revendication 1, 2, 3, 4 ou 5, selon lequel $R_3$ est le groupe isopropyle.

8. Un composé selon la revendication 1, 2, 3, 4 ou 5, selon lequel $R_3$ est un groupe aryloxyalkyle de formule

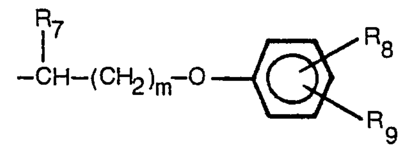

dans laquelle m, $R_7$, $R_8$ et $R_9$ sont comme définis dans la revendication 1.

9. Un composé selon la revendication 1, 2, 3, 4 ou 5, selon lequel $R_3$ est un groupe arylalkyle de formule

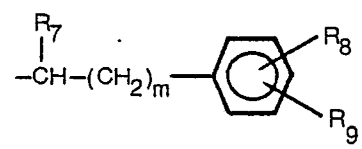

dans laquelle m, $R_7$, $R_8$ et $R_9$ sont comme définis dans la revendication 1.

10. Un composé selon la revendication 1, 2, 3, 4 ou 5, selon lequel $R_3$ est un groupe méthyle.

11. Un composé selon la revendication 1, 2, 3, 4 ou 5, selon lequel $R_3$ est un groupe benzodioxanne de formule

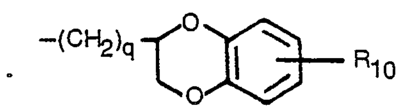

dans laquelle q et $R_{10}$ sont comme définis dans la revendication 1.

12. Une composition sous forme de dosage pharmaceutique comprenant le composé selon la revendication 1 et les sels pharmaceutiquement acceptables dudit composé, et un support ou diluant pharmaceutiquement acceptable.

**Revendications pour l'Etat contractant: AT**

1. Un procédé pour la préparation d'un composé de formule

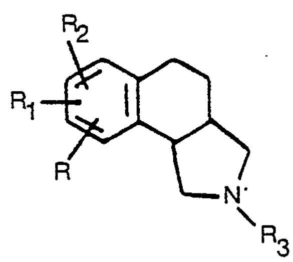

(I)

dans laquelle R, $R_1$ et $R_2$ sont choisis indépendamment parmi l'hydrogène, l'hydroxy, les groupes alcoxy de 1 à 3 atomes de carbone, où R et $R_1$, ou $R_1$ et $R_2$ peuvent être pris ensemble pour former un pont méthylènedioxy ou éthylènedioxy; à la condition qu'au moins l'un des R, $R_1$ ou $R_2$ doit être autre que l'hydrogène; et $R_3$ est l'hydrogène; un groupe alkyle de 1 à 4 atomes de carbone; un substituant de formule

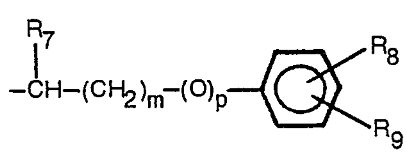

dans laquelle m est égal à 0, 1 ou 2, p est égal à 0 ou 1, $R_7$ est l'hydrogène ou un groupe alkyle de 1 à 4 atomes de carbone et $R_8$ et $R_9$ sont choisis indépendamment parmi l'hydrogène, l'hydroxy, le groupe méthoxy, les groupes alkyles de 1 à 4 atomes de carbone, ou halo, ou $R_8$ et $R_9$ peuvent être pris ensemble pour former un pont méthylènedioxy ou éthylènedioxy, ou un groupe 1,4-benzodioxanne de formule

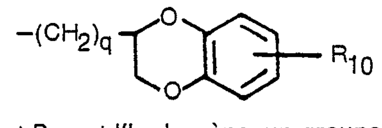

dans laquelle q est égal à 1, 2 ou 3, et $R_{10}$ est l'hydrogène, un groupe méthoxy ou halo; ainsi que les sels pharmaceutiquement acceptables dudit composé, comprenant

(i) la réduction d'un composé (4) dans lequel R', $R'_1$ et $R'_2$ sont choisis indépendamment parmi l'hydrogène et les groupes alcoxy de 1 à 3 atomes de carbone, l'un au moins des R', $R'_1$ et $R'_2$ étant autre que l'hydrogène, pour obtenir une 3a,4,5,9b-tétrahydro-benzo[e]isoindoline (I) dans laquelle $R_3$ est l'hydrogène et R, $R_1$ et $R_2$ ne sont pas des hydroxy,

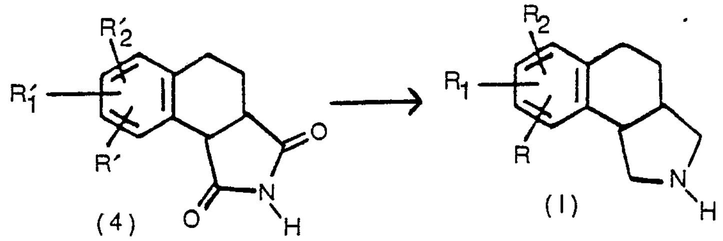

(4) (I)

(ii) la réaction de (I) avec un agent alkylant $R_3X$ pour obtenir un composé (I) dans lequel $R_3$ est différent de l'hydrogène ou la réaction de (I) avec un agent acylant choisi parmi $R_3$—COX et $R_3$—$SO_2X$ suivie de la réduction de l'amide résultant pour obtenir un composé (I) dans lequel $R_3$ est différent de l'hydrogène,

(iii) et éventuellement la désalkylation du composé (I) obtenu dans (ii) pour obtenir un composé (I) dans lequel R et/ou $R_1$ et/ou $R_2$ est un groupe hydroxy.

2. Le procédé selon la revendication 1, comprenant en outre une ou plusieurs étapes préliminaires de

(a) réaction d'une tétralone substituée (1) avec le cyanure de triméthylsilyle et ensuite avec HCl pour obtenir un 1-cyano-3,4-dihydronaphtalène (2)

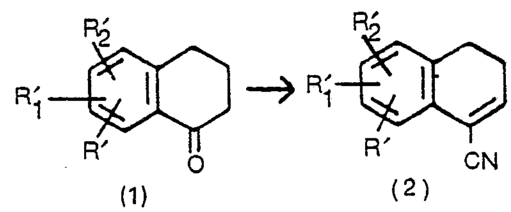

(1) (2)

(b) réaction de (2) avec un ion cyanure pour obtenir un 1,2-dicyano-1,2,3,4-tétrahydronaphtalène (3)

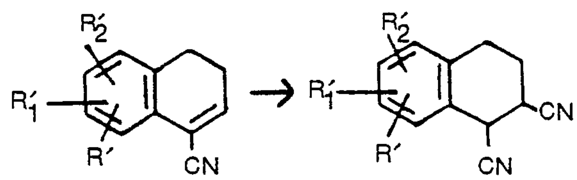

(2) (3)

(c) et traitement du composé (3) soit par l'acide sulfurique soit par l'acide bromhydrique gazeux et du DMF pour donner le composé (4)

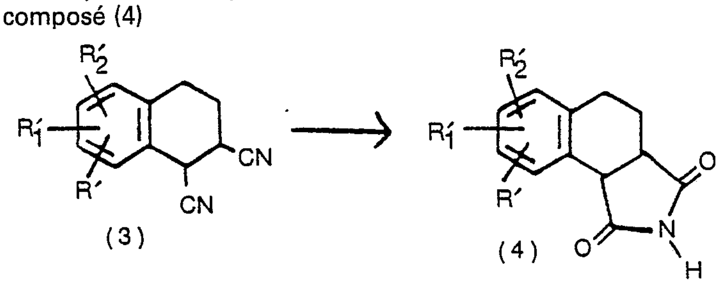

(3) (4)

3. Un procédé selon la revendication 1, pour préparer un composé I dans lequel l'un des R, $R_1$ et $R_2$ est l'hydrogène et les deux restants des R, $R_1$ et $R_2$ sont des groupes hydroxy.

4. Un procédé selon la revendication 1, pour préparer un composé I dans lequel l'un des R, $R_1$ et $R_2$ est l'hydrogène et les deux autres des R, $R_1$ et $R_2$ sont des groupes méthoxy.

5. Un procédé selon la revendication 1, pour préparer un composé I dans lequel R et $R_1$ sont pris ensemble pour former un groupe méthylènedioxy.

6. Un procédé selon la revendication 1, pour préparer un composé I dans lequel $R_1$ et $R_2$ sont pris ensemble pour former un groupe méthylènedioxy.

7. Un procédé selon la revendication 1, 2, 3, 4, 5 ou 6, pour préparer un composé I dans lequel $R_3$ est l'hydrogène.

8. Un procédé selon la revendication 1, 2, 3, 4, 5 ou 6, pour préparer un composé I dans lequel $R_3$ est un groupe isopropyle.

9. Un procédé selon la revendication 1, 2, 3, 4, 5 ou 6, pour préparer un composé I dans lequel $R_3$ est un groupe aryloxyalkyle de formule

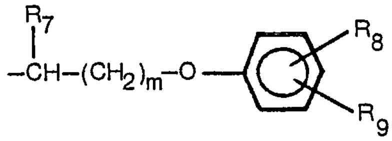

dans laquelle m, $R_7$, $R_8$ et $R_9$ sont comme définis dans la revendication 1.

10. Un procédé selon la revendication 1, 2, 3, 4, 5 ou 6, pour préparer un composé I dans lequel $R_3$ est un groupe arylalkyle de formule

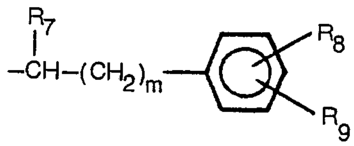

dans laquelle m, $R_7$, $R_8$ et $R_9$ sont comme définis dans la revendication 1 et dans laquelle $R_4$ est de l'hydrogène.

11. Un procédé selon la revendication 1, 2, 3, 4, 5 ou 6, pour préparer un composé I dans lequel $R_3$ est un groupe méthyle.

12. Un procédé selon la revendication 1, 2, 3, 4, 5 ou 6, pour préparer un composé I dans lequel $R_3$ est un groupe benzodioxanne de formule

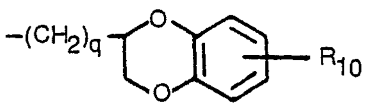

dans laquelle q et $R_{10}$ sont comme définis dans la revendication 1.

13. Un procédé pour préparer une composition sous forme de dosage pharmaceutique comprenant le mélange d'un composé préparé selon le procédé de la revendication 1 ou d'un sel pharmaceutiquement acceptable dudit composé avec un support ou diluant pharmaceutiquement acceptable.